# EUROPÄISCHE PATENTSCHRIFT

(11) **EP 2 762 094 B1**
(45) Veröffentlichungstag und Bekanntmachung des Hinweises auf die Patenterteilung: **20.09.2017**
(21) Anmeldenummer: 14152593.1
(22) Anmeldetag: 27.01.2014
(51) Int. Cl.: A61B 17/34

(54) **Dichteinrichtung zur Abdichtung einer Durchführung für ein medizinisches Instrument**
Sealing device for sealing a feedthrough for a medical instrument
Dispositif d'étanchéité pour étanchéifier un passage pour un instrument médical

(30) Priorität: 01.02.2013 DE 102013101019
(43) Veröffentlichungstag der Anmeldung: 06.08.2014
(73) Patentinhaber: Karl Storz GmbH & Co. KG, 78532 Tuttlingen (DE)
(72) Erfinder: Oberländer, Martin, 78532 Tuttlingen (DE); Wagner, Sebastian, 78532 Tuttlingen (DE)

(56) Entgegenhaltungen:
- DE-C1- 19 955 071
- US-A- 5 273 545
- US-A- 5 350 363
- US-A- 5 549 594
- US-A- 5 779 697
- US-A- 5 820 604
- US-A1- 2005 288 622
- US-A1- 2008 171 987
- US-A1- 2009 240 204

## Beschreibung

Die vorliegende Erfindung bezieht sich auf eine Dichteinrichtung zur Abdichtung einer Durchführung für ein medizinisches Instrument, insbesondere zur Abdichtung eines Tubus, durch den bei einem mikroinvasiven medizinischen Eingriff ein Endoskop und/oder andere medizinische Instrumente in eine natürliche oder künstliche Körperhöhle eingeführt werden können, oder eines Arbeitskanals eines Endoskops.

Ein Beispiel für mikroinvasive medizinische Methoden ist die Laparoskopie. Mittels eines Trokars wird ein künstlicher Zugang zur Bauchhöhle eines Patienten durch dessen Bauchdecke hindurch geschaffen. Durch das Lumen eines während des laparoskopischen Eingriffs in der Bauchdecke verbleibenden Tubus des Trokars können ein Endoskop und/oder weitere medizinische Instrumente (beispielsweise Zangen, Scheren, Nadelhalter) in die Bauchhöhle eingeführt werden. Während der Laparoskopie wird der Bauchraum mit Kohlenstoffdioxid oder einem anderen Gas gefüllt, um eine Pneumoperitoneum, einen den medizinischen Eingriff ermöglichenden Hohlraum zu schaffen. Ohne besondere Maßnahmen würde dieses Gas durch das Lumen des Tubus des Trokars entweichen. Deshalb wurden zahlreiche Ansätze entwickelt, um das Lumen des Tubus eines Trokars möglichst fluiddicht zu verschließen, und zwar sowohl im leeren Zustand als auch bei eingeführtem Instrument. Teilweise ähnliche Aufgabestellungen können sich an einem Arbeitskanal eines Endoskops oder beim Einführen eines Katheters in ein Blutgefäß ergeben. Im letztgenannten Fall ist jedoch nicht das Entweichen eines Gases, sondern von Blut zu verhindern.

In US 4,857,062 ist ein Ventil zum Einführen eines Katheters in eine Arterie beschrieben. Zur Abdichtung sind ein entenschnabelförmiges erstes Element und ein zweites flexibles Element, das zur Ausbildung einer fluiddichten Abdichtung mit einem Katheter komprimiert ist, vorgesehen. Beide Elemente sind hintereinander und starr in einem Gehäuse angeordnet.

In WO 93/01850 A1 ist eine durch Hebel betätigte Dichtung für einen Tubus beschrieben. Eine Wand aus einem Elastomer mit einer Öffnung wird beim Einführen eines Instruments in den Tubus durch mehrere Hebel gedehnt, wodurch die Öffnung vergrößert wird.

In US 5,366,446 ist eine Einführanordnung zur Verwendung auf der Haut eines Patienten beschrieben, die für das Einführen von Tuben mit unterschiedlichen Außendurchmessern ausgebildet ist. Die Anordnung umfasst eine Membran aus einem durchstechbaren elastomeren Material in der Mitte eines Faltenbalgs.

In EP 0 630 660 A1 ist eine Dichtanordnung zum Aufnehmen eines chirurgischen Instruments beschrieben. Die Dichtanordnung umfasst eine Entenschnabeldichtung oder eine Anordnung aus einer Mehrzahl von teilweise sternförmig geschlitzten Dichtelementen, die einander teilweise überlappen und teilweise konisch ausgebildet sind.

In EP 0 746 359 B1 ist ein Katheter-Sperrventil beschrieben. Zur Abdichtung sind eine Gummidichtung mit einer Öffnung und distal dieser ein Entenschnabelventil mit einem geraden Schlitz vorgesehen.

In WO 2010/045702 A1 ist eine Einwegdichtung für einen Tubus beschrieben. Die Einwegdichtung weist eine näherungsweise becherförmige Gestalt mit sich kreuzenden Schlitzen am Boden auf.

In US 4,430,081 ist eine Kanüle zur Verwendung mit Angiographiekathetern beschrieben. Zur Abdichtung gegen das Eindringen von Luft oder das Austreten von Blut aus einem Blutgefäß sind eine erste Dichtung mit einem Schlitz, eine zweite Dichtung mit einem Loch und eine dritte Dichtung mit einer Klappe darin vorgesehen, die aneinander angrenzend angeordnet sind.

In WO 91/12838 A1 ist ein Infusionszugang mit mehreren hintereinander angeordneten elastischen Scheiben, die jeweils kreisförmige Öffnungen oder gegeneinander verdreht angeordnete sternförmige Schlitze aufweisen, vorgesehen.

In EP 0 536 549 A1 ist eine Trokarhülse zum Durchführen eines medizinischen Instruments beschrieben. Eine Dichtungseinrichtung für eine Abdichtung eines Axialdurchgangs und eines Hohlschafts sowohl bei eingeführtem Instrument als auch bei nicht eingeführtem Instrument umfasst eine oder mehrere Trennwände aus elastischem Material mit gegeneinander versetzten Kreuzschlitzungen.

In WO 94/01149 A1 und DE 693 29 286 T2 ist ein Ventil für eine Einführungsanordnung beschrieben. Ein Körper aus Silikon oder einem anderen elastomeren Material umfasst eine zylindrische Wand, die eine Bohrung umschließt. Ein Ende der zylindrischen Wand und der Bohrung ist mit einer Wand mit einer zentral angeordneten Öffnung verschlossen. Das andere Ende der zylindrischen Wand und der Bohrung ist durch zwei gegeneinander geneigte Plättchen mit einem dazwischenliegenden Schlitz verschlossen.

In WO 98/32484 A1 ist ein Kathetereinführungsbesteck mit einem Hämostaseventil beschrieben. Ein Dichtelement umfasst zwei gelochte Stützscheiben, zwischen denen eine Dichtscheibe aus weich-elastischem Schaumkunststoff mit radial verlaufenden Schlitzen vorgesehen ist.

In EP 1 269 925 A1 ist eine Zugangskanüle für endoskopische Operationen beschrieben. Ein Doppelscheibenventil umfasst zwei Scheiben mit je einem dreistrahlig sternförmigen Schlitz, wobei die Schlitze der beiden Scheiben gegeneinander verdreht angeordnet sind.

In EP 1 350 476 A1 ist eine Trokarhülse mit einem Ventil beschrieben. Das Ventil umfasst einen Einführbereich mit in distaler Richtung aufeinander zulaufenden, als Schrägflächen ausgebildeten Wandabschnitten und elastisch aneinander anliegenden Dichtlippen.

In US 5,820,604 ist eine Kappe für einen Tubus eines Trokars beschrieben. Die zur Aufnahme eines medizinischen Instruments vorgesehene Öffnung ist umgeben von einer Falte mit inneren und äußeren Wandabschnitten, die an ihren oberen Enden miteinander verbunden sind. Dieses Dokument wird als nächstliegender Stand der Technik angesehen. Die beschriebenen Dichteinrichtungen weisen jeweils spezifische Vor- und Nachteile auf. Für viele Anwendungen sind noch keine befriedigenden Lösungen gefunden oder zumindest weitere Verbesserungen wünschenswert. Dies gilt insbesondere im Hinblick darauf, dass eine Dichteinrichtung zahlreiche Anforderungen gleichzeitig erfüllen soll. Dazu zählt, dass die Dichteinrichtung fluiddicht, robust, wiederverwendbar und dazu insbesondere autoklavierbar sein, einer Bewegung eines medizinischen Instruments in axialer Richtung möglichst geringen Widerstand entgegensetzen, eine Hebelmanipulation bzw. ein Kippen eines in die Dichteinrichtung eingesetzten medizinischen Instruments ermöglichen und auch dabei fluiddicht bleiben, weder beim Einführen noch beim Herausziehen eines medizinischen Instruments sich umstülpen sowie kostengünstig herstellbar sein soll. Eine Aufgabe der vorliegenden Erfindung besteht darin, eine verbesserte Dichteinrichtung zu schaffen, die insbesondere die aufgezählten Anforderungen und Erwartungen in ausgewogenen Verhältnissen erfüllt.

Diese Aufgabe wird durch die Gegenstände der unabhängigen Ansprüche gelöst. Weiterbildungen sind in den abhängigen Ansprüchen angegeben.

Eine Dichteinrichtung zur Abdichtung einer Durchführung für ein medizinisches Instrument umfasst einen Befestigungsbereich zur Befestigung der Dichteinrichtung an einem Tubus oder einer anderen medizinischen Vorrichtung; einen Dichtbereich, der zumindest entweder ausgebildet ist, um am Umfang eines in die Dichteinrichtung eingeführten medizinischen Instruments anzuliegen, oder ausgebildet ist, um die Dichteinrichtung zu verschließen, wenn kein medizinisches Instrument in die Dichteinrichtung eingeführt ist; eine erste elastische Wand, die den Dichtbereich ringartig umschließt, mit einem ringförmigen ersten Rand, der mit dem Dichtbereich verbunden ist, und einem ringförmigen zweiten Rand; eine zweite elastische Wand, die die erste Wand ringartig umgibt und von dieser beabstandet ist, mit einem ringförmigen ersten Rand und einem ringförmigen zweiten Rand, der mit dem Befestigungsbereich verbunden ist; einen ringförmigen Übergangsbereich, der den zweiten Rand der ersten Wand mit dem ersten Rand der zweiten Wand verbindet, wobei der Übergangsbereich eine größere Wanddicke aufweist als die erste Wand und als die zweite Wand.

Die Dichteinrichtung ist insbesondere eine Dichteinrichtung für einen Tubus eines Trokars, der während eines mikroinvasiven Eingriffs einen Zugang zu einem Hohlraum bildet. Alternativ ist die Dichteinrichtung vorgesehen und ausgebildet für einen Arbeitskanal eines Endoskops oder für eine andere medizinische Vorrichtung, in die ein medizinisches Instrument eingeführt werden kann, und die mit und ohne das medizinische Instrument fluiddicht verschlossen sein soll. Der Dichtbereich ist insbesondere am proximalen Ende oder an der proximalen Seite der Dichteinrichtung vorgesehen.

Der Befestigungsbereich ist insbesondere am distalen Ende oder an der distalen Seite der Dichteinrichtung vorgesehen. Der Befestigungsbereich ist insbesondere zur lösbaren Befestigung der Dichteinrichtung am proximalen Ende eines Tubus oder eines Arbeitskanals eines Endoskops oder einer anderen medizinischen Vorrichtung vorgesehen und ausgebildet. Dazu umfasst der Befestigungsbereich insbesondere einen elastischen Kragen oder einen anderen Oberflächenabschnitt, der eine formschlüssige mechanische Verbindung mit einem korrespondierend ausgebildeten Oberflächenabschnitt an der medizinischen Vorrichtung ermöglicht. Insbesondere umfasst die Dichteinrichtung einen ringförmigen, nach innen ragenden Kragen, der dazu ausgebildet ist, in eine ringförmige, nach außen offene Nut an einer medizinischen Vorrichtung einzugreifen, oder eine ringförmige, nach innen offene Nut, die dazu ausgebildet ist, einen nach außen ragenden Kragen an einer medizinischen Vorrichtung aufzunehmen.

Die erste und die zweite Wand sind jeweils beispielsweise im Wesentlichen kreisringförmig, elliptisch oder polygonal. Insbesondere weisen die erste und die zweite Wand jeweils im Wesentlichen die Gestalt eines ringförmigen Ausschnitts einer Mantelfläche eines Zylinders oder eines Kegels mit kreisförmiger, elliptischer, polygonaler oder anderer Grundfläche bzw. Querschnitt auf.

Die zweite Wand ist insbesondere von der ersten Wand radial beabstandet. Die erste Wand und die zweite Wand sind insbesondere parallel oder im Wesentlichen (Winkel nicht größer als 10 Grad oder nicht größer als 20 Grad) parallel zueinander. Sowohl die erste als auch die zweite elastische Wand kann jeweils eine konstante Wanddicke oder eine von proximal nach distal zunehmende oder abnehmende Wanddicke aufweisen.

Der erste Rand und der zweite Rand jeder Wand sind jeweils insbesondere kreisförmig. Der erste Rand der ersten Wand ist insbesondere unmittelbar mit der Ringdichtung und unmittelbar oder über die Ringdichtung mittelbar mit dem Rand der geschlitzten Membran verbunden. Der zweite Rand der ersten Wand kann unmittelbar oder mittelbar mit dem Befestigungsbereich verbunden sein. Insbesondere verbindet der ringförmige Übergangsbereich den zweiten Rand der ersten Wand und den ersten Rand der zweiten Wand in radialer Richtung.

Die Wanddicke ist insbesondere der Abstand zwischen zwei einander gegenüberliegenden Punkten an den beiden von einander abgewandten Oberflächen der Wand. Zwei Punkte liegen insbesondere und zumindest in einfachen Fällen dann einander gegenüber, wenn eine Gerade durch beide Punkte senkrecht zu mindestens einer der beiden von einander abgewandten Oberflächen der Wand ist.

Die im Übergangsbereich erhöhte Wanddicke der Dichteinrichtung bewirkt eine erhöhte Steifigkeit des Übergangsbereichs. Diese erhöhte Steifigkeit im Übergangsbereich reduziert Verformungen der Dichteinrichtung im Übergangsbereich. Eine erhöhte Steifigkeit des Übergangsbereichs reduziert das Risiko eines Umstülpens ausgehend vom Übergangsbereichs. Ein medizinisches Instrument in der Dichteinrichtung kann deshalb mit einem geringeren Risiko eines Umstülpens in beide Richtungen bewegt werden, nämlich sowohl nach distal als auch nach proximal.

Bei einer Dichteinrichtung, wie sie hier beschrieben ist, ist die größte Wanddicke des Übergangsbereichs insbesondere um mindestens 50 Prozent größer als die minimale Wanddicke der zweiten Wand.

Die größte Wanddicke des Übergangsbereichs beträgt somit insbesondere das 1,5-fache der minimalen Wanddicke der zweiten Wand. Insbesondere beträgt die größte Wanddicke des Übergangsbereichs das 1,5-fache bis 2-fache und oder das 1,6-fache bis 1,8-fache der minimalen Wanddicke der zweiten Wand. Die minimale Wanddicke der zweiten Wand liegt insbesondere nahe dem Übergangsbereich oder an der Grenze zwischen der zweiten Wand und dem Übergangsbereich vor.

Bei einer Dichteinrichtung, wie sie hier beschrieben ist, nimmt die Wanddicke der ersten Wand insbesondere vom Dichtbereich zum Übergangsbereich zu.

Insbesondere nimmt die Wanddicke der ersten Wand kontinuierlich bzw. als stetige oder sogar als streng monoton steigende Funktion des Orts vom Dichtbereich zum Übergangsbereich zu.

Bei einer Dichteinrichtung, wie sie hier beschrieben ist, nimmt die Dicke der zweiten Wand vom Übergangsbereich zum Befestigungsbereich zu.

Insbesondere nimmt die Wanddicke der zweiten Wand kontinuierlich bzw. als stetige oder sogar als streng monoton steigende Funktion des Orts vom Übergangsbereich zum Befestigungsbereich zu.

Eine kontinuierliche Zunahme der Wanddicke und insbesondere Variation der Wanddicke als streng monoton Funktion des Orts kann nicht nur fertigungstechnisch vorteilhaft sein, sondern auch mechanische Spannungsspitzen bei einer Verformung der Dichteinrichtung vermeiden.

Bei einer Dichteinrichtung, wie sie hier beschrieben ist, weist der Übergangsbereich insbesondere in einer Schnittebene, die eine Symmetrieachse der ersten Wand und der zweiten Wand enthält, einen im Wesentlichen bogenförmigen Querschnitt auf.

Der Querschnitt ist insbesondere im Wesentlichen halbkreisbogenförmig. Eine im Querschnitt bogenförmige Ausgestaltung des Übergangsbereichs vermeidet lokale mechanische Spannungsspitzen im Falle einer Verformung der Dichteinrichtung und verbessert so die mechanische Robustheit und die Lebensdauer der Dichteinrichtung.

Bei einer Dichteinrichtung, wie sie hier beschrieben ist, umfasst der Dichtbereich insbesondere eine geschlitzte Membran zum Verschließen der Dichteinrichtung, wenn kein medizinisches Instrument in die Dichteinrichtung eingeführt ist, und eine Ringdichtung, die ausgebildet ist, um am Umfang eines in die Dichteinrichtung eingeführten medizinischen Instruments anzuliegen, wobei ein äußerer Rand der geschlitzten Membran bezogen auf die Einführrichtung, in der ein medizinisches Instrument in die Dichteinrichtung einzuführen ist, unmittelbar hinter der Ringdichtung angeordnet.

Die Ringdichtung ist insbesondere hinsichtlich ihrer geometrischen Eigenschaften an einen Schaft eines vorbestimmten medizinischen Instruments oder an einen vorbestimmten Schaftquerschnitt eines medizinischen Instruments angepasst. Die Ringdichtung kann ausgebildet sein, um bereits im entspannten Zustand bzw. wenn kein medizinisches Instrument in die Dichteinrichtung eingesetzt ist, abschnittsweise eine einem Torus ähnliche Gestalt auszuweisen. Alternativ kann die Ringdichtung bei in die Dichteinrichtung eingesetztem medizinischen Instrument abschnittsweise eine einem Torus ähnliche Gestalt ausweisen. Insbesondere ist die Ringdichtung ausgebildet, um bei in die Dichteinrichtung eingesetztem medizinischen Instrument zumindest in ihrem an das medizinische Instrument angrenzenden Bereich hinsichtlich eines Schnitts entlang einer Ebene, die die Längsachse des medizinischen Instruments enthält, einen konvexen Querschnitt aufzuweisen.

Die Ringdichtung und die geschlitzte Membran sind insbesondere ausgebildet, um gemeinsam einen im Wesentlichen becherförmigen Oberflächenabschnitt der Dichteinrichtung zu bilden, wenn kein medizinisches Instrument in die Dichteinrichtung eingesetzt ist.

Die geschlitzte Membran weist nach proximal und nach distal insbesondere zwei parallele oder im Wesentlichen parallele Oberflächen auf. Die geschlitzte Membran ist insbesondere eben oder im Wesentlichen eben. Die geschlitzte Membran weist insbesondere einen Schlitz mit drei oder mehr radial verlaufenden Abschnitten mit gleichen oder im Wesentlichen gleichen Winkelabständen auf.

Die Merkmale der Dichteinrichtung können ein fludidichtes Verschließen eines Tubus oder einer anderen medizinischen Vorrichtung durch die geschlitzte Membran, wenn kein medizinisches Instrument in die Dichteinrichtung eingeführt ist, und durch die Ringdichtung, wenn ein medizinisches Instrument in die Dichteinrichtung eingeführt ist, ermöglichen. Indem sowohl die Ringdichtung als auch die geschlitzte Membran am gleichen ersten Rand der ersten Wand angeordnet und über die erste Wand und die zweite Wand mit dem Befestigungsbereich verbunden sind, wirken aufgrund der Elastizität der ersten Wand und der zweiten Wand auch bei einer Verschiebung des medizinischen Instruments in einer Richtung senkrecht zur Einführrichtung keine wesentlich erhöhten Kräfte zwischen dem medizinischen Instrument einerseits und der Ringdichtung und der geschlitzten Membran andererseits. Die Dichtwirkung der geschlitzten Membran und vor allem der am Umfang des medizinischen Instruments anliegenden Ringdichtung wird deshalb durch Kipp- oder Hebelbewegungen des medizinischen Instruments nicht oder nicht wesentliche beeinflusst. Die Fluiddichtheit der Dichteinrichtung kann somit auch bei einem praktischen Gebrauch gewährleistet sein, bei dem die Dichtwirkung anderer Dichteinrichtungen durch Kräfte zwischen dem Instrument und der Dichteinrichtung gefährdet oder beeinträchtigt ist.

Die Einführrichtung ist insbesondere senkrecht zum ersten Rand und senkrecht zum zweiten Rand der ersten Wand.

Die Elastizität der ersten Wand und der zweiten Wand und die daraus resultierenden vergleichsweise geringen Kräfte zwischen einem in die Dichteinrichtung eingesetzten medizinischen Instrument einerseits und der geschlitzten Membran und der Ringdichtung andererseits können ferner das Risiko einer Beschädigung der Ringdichtung oder der geschlitzten Membran senken. Die Dichteinrichtung kann deshalb eine besonders hohe Zuverlässigkeit und eine besonders lange Lebensdauer aufweisen. Eine wiederverwendbare Ausgestaltung der Dichteinrichtung kann deshalb sinnvoll sein und zu niedrigen Lebenszykluskosten beitragen.

Eine Anordnung der geschlitzten Membran in unmittelbarer Nähe der Ringdichtung kann die beschriebene Wirkung der Elastizität der ersten Wand unterstützen. Nicht nur bei einer Verschiebung eines medizinischen Instruments in einer Richtung senkrecht zur Einführrichtung, sondern auch bei einer Kippung des medizinischen Instruments um eine Achse senkrecht zur Einführrichtung resultieren in diesem Fall nur vergleichsweise geringe Kräfte auf die Ringdichtung und die geschlitzte Membran. Eine Anordnung der geschlitzten Membran in Einführrichtung hinter der Ringdichtung ermöglicht eine dichtende Wirkung der am Umfang eines medizinischen Instruments anliegenden Ringdichtung bereits bevor das medizinische Instrument die geschlitzte Membran öffnet.

Eine einstückige Ausbildung von Ringdichtung und geschlitzter Membran (insb. an einem einzigen Gussteil) kann die Fertigungskosten reduzieren, eine geringere Baugröße ermöglichen und die mechanische Robustheit der Dichteinrichtung verbessern.

Eine Dichteinrichtung, wie sie hier beschrieben ist, umfasst insbesondere ferner eine Verstärkung am Ende eines Schlitzes in der Dichtmembran.

Die Verstärkung ist beispielsweise in Form einer lokal vergrößerten Wanddicke bzw. Materialstärke der Dichtmembran im Bereich des Endes eines Schlitzes ausgestaltet. Eine derartige Verstärkung kann das Risiko eines Einreißens der Dichtmembran an einem Ende des Schlitzes senken und damit die Robustheit und die Lebensdauer der Dichteinrichtung steigern.

Eine Dichteinrichtung, wie sie hier beschrieben ist, umfasst insbesondere ferner eine Lasche nahe dem Befestigungsbereich, zum manuellen Lösen einer Befestigung der Dichteinrichtung an einem Tubus oder einer anderen medizinischen Vorrichtung.

Die Lasche vereinfacht insbesondere ein einfaches, sicheres und ergonomisches Greifen der Dichteinrichtung beim manuellen Verbinden der Dichteinrichtung mit einem Tubus oder einer anderen medizinischen Vorrichtung und vor allem beim manuellen Trennen der Dichteinrichtung von dem Tubus oder der anderen medizinischen Vorrichtung. Die Anordnung der Lasche an oder nahe dem Befestigungsbereich der Dichteinrichtung kann bewirken, dass eine manuell erzeugte Zugkraft sehr unmittelbar auf den Befestigungsbereich wirkt. So kann auf einfache und sichere Weise beispielsweise ein nach innen ragender Kragen an der Dichteinrichtung aus einer nach außen offenen Nut an einer medizinisehen Vorrichtung gezogen werden, um eine mechanische Verbindung von Dichteinrichtung und medizinischer Vorrichtung zu trennen.

Eine Dichteinrichtung, wie sie hier beschrieben ist, ist insbesondere einstückig ausgebildet.

Insbesondere ist die Dichteinrichtung als Gussteil aus Silikon oder einem anderen elastischen, biokompatiblen und autoklavierbaren Material ausgebildet. Eine einstückige Ausbildung der Dichteinrichtung kann eine einfache Zerlegung und Reinigung der Dichteinrichtung unterstützen. Eine einstückige Ausbildung der Dichteinrichtung kann ferner im Vergleich zu einer mehrstückigen Ausgestaltung geringere Kosten für Herstellung, Lagerhaltung und Logistik ermöglichen.

Eine Dichteinrichtung, wie sie hier beschrieben ist, umfasst insbesondere ferner eine reibungsmindernde Schicht an einem Oberflächenbereich, der ausgebildet ist, um ein in die Dichteinrichtung eingeführtes medizinisches Instrument zu berühren.

Insbesondere ist eine reibungsmindernde Schicht an der gesamten Oberfläche der Dichteinrichtung oder zumindest im Bereich der Ringdichtung und/oder der geschlitzten Membran vorgesehen. Eine reibungsmindernde Schicht kann die beim Einführen eines Instruments in die Dichteinrichtung und beim Herausziehen des Instruments aus der Dichteinrichtung wirkenden Kräfte vermindern. Die Schicht kann sowohl die Haftreibung als auch die Gleitreibung herabsetzen. Eine Minderung der Gleit- und/oder Haftreibung kann eine feinfühligere Bewegung eines medizinischen Instruments in der Dichteinrichtung ermöglichen und eine mechanische Belastung der Dichteinrichtung vermindern. Eine verminderte mechanische Belastung der Dichteinrichtung kann das Risiko eines Umstülpens der ersten Wand und das Risiko einer Beschädigung der Dichteinrichtung, beispielsweise eines Einreißens, mindern.

Die reibungsmindernde Schicht umfasst insbesondere Poly(p-Xylylene), das auch unter dem Markennamen Parylene vertrieben wird.

Die Dichteinrichtung weist insbesondere einen oder mehrere über den Umfang der ersten Wand gleichmäßig verteilte Stege auf, die zumindest teilweise in Richtung vom ersten Rand zum zweiten Rand der ersten Wand ausgerichtet sind. Der Steg oder jeder einzelne der Stege ist zumindest entweder mit der ersten Wand oder mit der zweiten Wand verbunden. Die Stege reichen insbesondere vom ersten Rand bis zum zweiten Rand der ersten Wand. Der Steg oder die Stege erstrecken sich insbesondere über einen Teil des Zwischenraums zwischen der ersten Wand und der zweiten Wand.

Die erste Wand, die zweite Wand und der oder die Stege sind insbesondere derart elastisch verformbar, dass eine Verschiebung des Dichtbereichs relativ zum Befestigungsbereich in Richtungen senkrecht zu der Richtung, in der ein medizinisches Instrument in die Dichteinrichtung eingeführt werden kann, möglich ist. Ferner können die erste Wand, die zweite Wand und der oder die Stege derart elastisch verformbar, dass sie ein Kippen oder Schwenken der Ringdichtung und der geschlitzten Membran relativ zum Befestigungsbereich ermöglichen.

Der oder die Stege sind zumindest abschnittsweise parallel zur Einführrichtung angeordneter bzw. ausgerichtet. Altnernativ oder zusätzlich sind der oder die Stege zumindest abschnittsweise helikal zur Einführrichtung ausgebildet. Ferner können der oder die Stege radial oder zumindest teilweise tangential zur ersten Wand und/oder zur zweiten Wand ausgebildet sein.

Die erste Wand und die zweite Wand bilden insbesondere eine Falte eines (planaren) Faltenbalgs. Es kann vorteilhaft sein, dass die zweite Wand eine geringere Elastizität aufweist als die erste Wand. Dazu weist die zweite Wand insbesondere eine größere Wanddicke auf als die erste Wand.

Ein Tubus umfasst eine Dichteinrichtung, wie sie hier beschrieben ist.

Der Tubus ist insbesondere der Tubus bzw. die Hülse eines Trokars, der bzw. die nach dem Erzeugen einer Öffnung oder eines Zugangs in dieser verbleibt. Der Tubus weist insbesondere außer der Dichteinrichtung keine weitere Dichteinrichtung zum Verschluss des Tubus bei in den Tubus eingesetzten medizinischem Instrument oder zum Verschluss des Tubus ohne eingesetztes medizinisches Instrument auf.

### Kurzbeschreibung der Figuren

Nachfolgend werden Ausführungsformen anhand der beigefügten Figuren näher erläutert. Es zeigen:
- Figur 1: eine schematische Darstellung eines Tubus eines Trokars mit einer Dichteinrichtung und einem medizinischen Instrument;
- Figur 2: eine weitere schematische Darstellung des Tubus mit der Dichteinrichtung und dem medizinischen Instrument;
- Figur 3: eine schematische axonometrische Darstellung eines weiteren Tubus mit einer weiteren Dichteinrichtung;
- Figur 4: eine schematische Schnittdarstellung der Dichteinrichtung aus Figur 3;
- Figur 5: eine weitere schematische Schnittdarstellung der Dichteinrichtung aus den Figuren 3 und 4;
- Figur 6: eine weitere schematische Darstellung der Dichteinrichtung aus den Figuren 3 bis 5.

### Beschreibung der Ausführungsformen

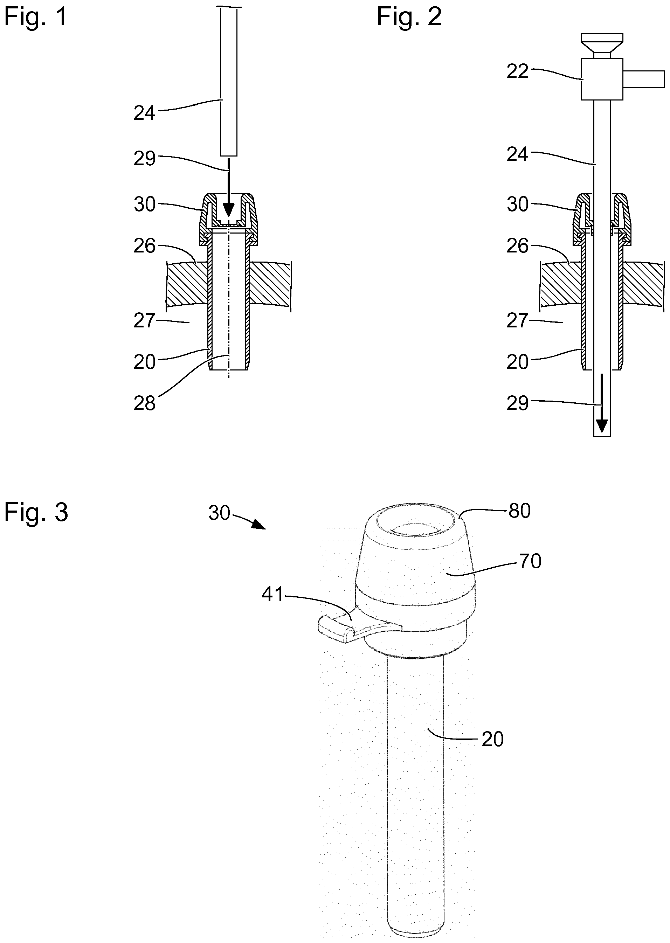

Die Figuren 1 und 2 zeigen schematische Schnittdarstellungen eines Tubus 20 eines Trokars, der in eine Bauchdecke 26 eines Patienten eingesetzt ist. Durch das Lumen des Tubus 20 hindurch können ein oder mehrere medizinische Instrumente in einen Hohlraum 27 unter der Bauchdecke 26 des Patienten eingeführt werden. Der Tubus 20, die Bauchdecke 26 und der Hohlraum 27 unter der Bauchdecke 26 sind in einem Schnitt entlang einer Ebene, die eine Längsachse 28 des Tubus 20 enthält, dargestellt. Der Tubus 20 ist insbesondere zumindest teilweise rotationssymmetrisch zur Längsachse 28. Der Tubus 20 umfasst eine Dichteinrichtung 30 an seinem proximalen Ende außerhalb des Hohlraums 27. Ausführungsformen der Dichteinrichtung 30 sind mit Bezug auf die Figuren 3 bis 8 näher beschrieben.

Ferner ist in den Figuren 1 und 2 ein medizinisches Instrument 22 mit einem Schaft 24 dargestellt. Da der innere Aufbau des medizinischen Instruments 22 und von dessen Schaft 24 für die nachfolgend beschriebenen Eigenschaften des Tubus 20 unerheblich sind, sind der Einfachheit halber nur Konturen des medizinischen Instruments 22 und von dessen Schaft 24 dargestellt. Das medizinische Instrument 22 ist in Figur 2 beispielhaft als Endoskop dargestellt.

Der Schaft 24 des medizinischen Instruments 22 kann in einer Richtung 29 parallel oder im Wesentlichen parallel zur Längsachse 28 des Tubus 20 in den Tubus 20 eingeführt werden. Der Schaft 24 des medizinischen Instruments 22 ist in Figur 1 vor dem Einführen in den Tubus 20 bzw. in dessen Lumen und in Figur 2 nach dem Einführen in den Tubus 20 bzw. dessen Lumen dargestellt. Die Dichteinrichtung 30 verschließt das proximale Ende des Tubus 20 sowohl dann, wenn, wie in Figur 1 dargestellt, kein medizinisches Instrument in den Tubus 20 eingeführt ist, als auch dann, wenn, wie in Figur 2 dargestellt, ein medizinisches Instrument 22 in den Tubus 20 eingeführt ist, fluiddicht. Dadurch verhindert die Dichteinrichtung 30 einen Austritt eines Gases oder eines anderen Fluids aus dem Hohlraum 27 unter der Bauchdecke 26 des Patienten.

Figur 3 zeigt eine schematische axonometrische Darstellung eines weiteren Ausführungsbeispiels eines Tubus 20 mit einer Dichteinrichtung 30. Die Dichteinrichtung 30 des Ausführungsbeispiels aus Figur 3 unterscheidet sich von der anhand der Figuren 1 und 2 dargestellten Dichteinrichtung insbesondere durch eine Lasche 41 nahe einem proximalen Ende der Dichteinrichtung 30. Die oben anhand der Figuren 1 und 2 dargestellte Dichteinrichtung kann abgesehen von der Lasche ähnliche oder identische Eigenschaften aufweisen wie die nachfolgend anhand der Figuren 3 bis 6 dargestellte Dichteinrichtung.

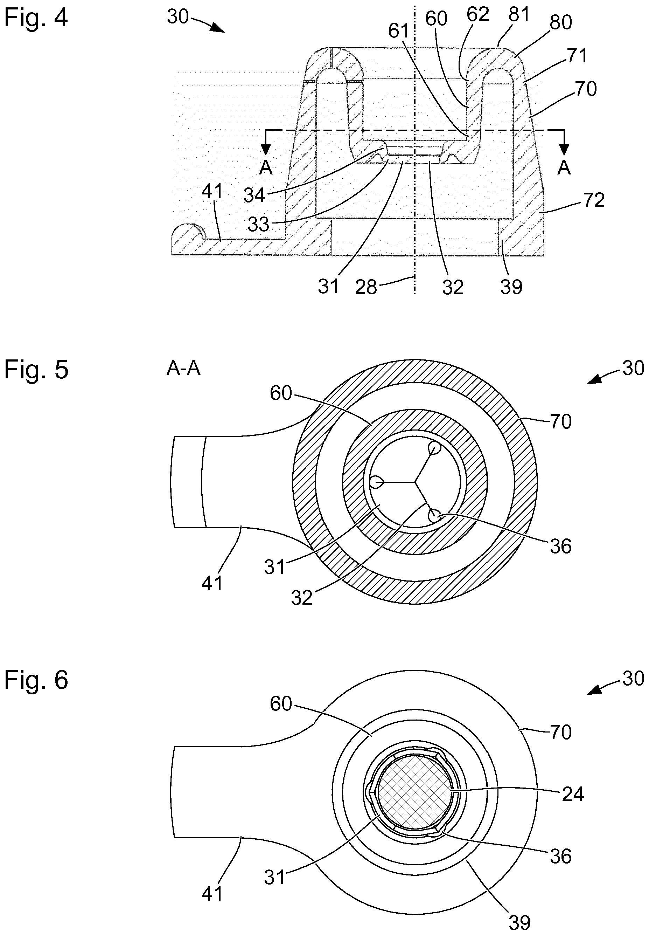

Figur 4 zeigt eine schematische Schnittdarstellung der Dichteinrichtung aus Figur 3. Die Dichteinrichtung 30 ist entlang einer Ebene, die die Längsachse 28 des Tubus 20 enthält, aufgeschnitten dargestellt.

Die Dichteinrichtung 30 ist einstückig ausgebildet, insbesondere aus Silikon oder einem anderen elastischen Material gegossen. Die Dichteinrichtung 30 umfasst eine Dichtmembran 31. Die Dichtmembran 31 ist im Wesentlichen kreisförmig und symmetrisch zur Längsachse 28 angeordnet. Die Dichtmembran 31 weist proximal und distal zwei im Wesentlichen ebene und parallele Seiten bzw. Oberflächen auf. Ferner weist die Dichtmembran 31 einen Schlitz 32 aus drei im Wesentlichen symmetrisch zueinander und radial angeordneten Abschnitten auf, von denen einer in der Schnittebene der Figur 3 liegt (rechts der Längsachse 28).

Der kreisförmige Rand 33 der Dichtmembran 31 geht in einen als Ringdichtung 34 ausgebildeten Bereich der Dichteinrichtung 30 über. Der die Ringdichtung 34 bildende Bereich der Dichteinrichtung 30 ist im Wesentlichen kreisringförmig und weist einen Querschnitt auf, der eine geeignete Elastizität der Ringdichtung 34 bewirkt. Die Dichtmembran 31 und die Ringdichtung 34 bilden in dem in Figur 3 dargestellten entspannten Zustand der Dichteinrichtung 30 einen Dichtbereich in der Form eines flachen Bechers, dessen Boden die Dichtmembran 31 und dessen Wand und Rand die Ringdichtung 34 bilden.

Die Ringdichtung 34 geht nach radial außen in eine erste elastische Wand 60 über, die im Wesentlichen die Gestalt eines ringförmigen Ausschnitts eines Kreiszylindermantels oder eines Kegelmantels aufweist. Ein erster Rand 61 der ersten Wand 60 ist mit der Ringdichtung 34 verbunden.

Die Dichtmembran 31, die Ringdichtung 34 und die erste elastische Wand 60 der Dichteinrichtung 30 sind von einer zweiten elastischen Wand 70 im Wesentlichen ringartig umgeben. Die zweite Wand 70 weist im Wesentlichen die Gestalt eines ringförmigen Abschnitts eines Kreiszylindermantels oder eines Kegelmantels auf. Ein Übergangsbereich 80 verbindet den zweiten Rand 62 der ersten Wand 60 mit einem ersten Rand 71 der zweiten Wand 70. Ein zweiter Rand 72 der zweiten Wand 70 geht in ein Randprofil 39 in Form eines nach radial innen zur Längsachse 28 hin ragenden Kragens über.

Die Richtung vom ersten Rand 61 der ersten Wand 60 bzw. vom Dichtbereich 31, 34 einerseits zum zweiten Rand 62 der ersten Wand bzw. zum Übergangsbereich 80 andererseits ist entgegengesetzt zu der Richtung vom ersten Rand 71 der zweiten Wand 70 bzw. vom Übergangsbereich 80 einerseits zum zweiten Rand 72 der zweiten Wand 70 bzw. zum Randprofil 39 andererseits. Dadurch liegt der Dichtbereich 31, 34 näher beim Randprofil 39 als der Übergangsbereich 80. Die erste Wand 60, der Übergangsbereich 80 und die zweite Wand 70 bilden eine im Querschnitt U-förmige Struktur.

Die Wanddicke der ersten Wand 60 nimmt bei dem dargestellten Beispiel vom ersten Rand 61 bzw. vom Dichtbereich 31, 34 zum zweiten Rand 62 bzw. zum Übergangsbereich 80 zu. Die Wanddicke der zweiten Wand 70 nimmt bei dem dargestellten Beispiel vom ersten Rand 71 bzw. vom Übergangsbereich 80 zum zweiten Rand 72 zu. Sowohl die Wanddicke der ersten Wand 60 als auch die Wanddicke der zweiten Wand 70 ist bei dem dargestellten Beispiel jeweils eine monotone, insbesondere affin-lineare Funktion des Orts bzw. einer parallel zur Längsachse 28 gemessenen Koordinate.

Die Elastizität der Wände 60, 70 ermöglicht eine Bewegung des aus Dichtmembran 31 und Ringdichtung 34 gebildeten Dichtbereichs einerseits gegenüber dem durch das Randprofil 39 gebildeten Befestigungsbereich andererseits, insbesondere in Richtungen senkrecht zur Längsachse 28. Durch die Auswahl des Materials der Dichteinrichtung 30 und der Wanddicken der Wände 60, 70 ist die Elastizität der Wände 60, 70 in weiten Bereichen einstellbar.

Der Übergangsbereich 80 zwischen der ersten Wand 60 und der zweiten Wand 70 weist in der in Figur 4 gezeigten Schnittebene, die die Längsachse 28 enthält, im Wesentlichen die Gestalt eines Halbkreisbogens auf. Die Breite dieses Halbkreisbogens bzw. die Wanddicke des Übergangsbereichs variiert. Ausgehend vom zweiten Rand 62 der ersten Wand 60 nimmt die Wanddicke der Dichtungseinrichtung 30 im Übergangsbereich 80 zunächst bis zu einer maximalen Wanddicke zu und dann bis zum ersten Rand 71 der zweiten Wand 70 hin wieder ab.

Die maximale Wanddicke liegt bei oder nahe dem Scheitel 81 des Übergangsbereichs 80 vor und beträgt insbesondere zwischen ca. 115 Prozent und ca. 135 Prozent (ca. 5/4) der Wanddicke der ersten Wand 60 nahe deren zweitem Rand 62 und zwischen ca. 140 Prozent und ca. 180 Prozent (ca. 5/3 der Wanddicke der zweiten Wand 70 nahe deren erstem Rand 71. Bei dem dargestellten Beispiel variiert die Wanddicke sowohl innerhalb des Übergangsbereichs 80 als auch zwischen dem Übergangsbereichen 80 und dem zweiten Rand 62 der ersten Wand 60 und zwischen dem Übergangsbereich 80 und dem ersten Rand 71 der zweiten Wand 70 jeweils kontinuierlich.

Abweichend von der Darstellung in Figur 4 kann die Wanddicke der Dichteinrichtung 30 sowohl im Bereich der ersten Wand 60 als auch im Bereich der zweiten Wand 70 als auch im Übergangsbereich 80 jeweils diskontinuierlich bzw. stufenförmig variieren. Ferner kann die Wanddicke zwischen der ersten Wand 60 und dem Übergangsbereich und/oder zwischen der zweiten Wand 60 und dem Übergangsbereich 80 stufenförmig variieren bzw. sich ändern.

Ferner kann abweichend von der Darstellung in Figur 4 der Übergangsbereich 80 eine nicht-bogenförmige Gestalt aufweisen. Beispielsweise kann der Übergangsbereich einen rechteckigen oder im Wesentlichen rechteckigen oder einen kreisförmigen oder im Wesentlichen kreisförmigen Querschnitt aufweisen. In jedem Fall bewirkt eine im Übergangsbereich 80 erhöhte Wanddicke eine erhöhte mechanische Steifigkeit des Übergangsbereichs 80 und reduziert dadurch die Wahrscheinlichkeit eines vom Übergangsbereich 80 ausgehenden Umstülpens.

Abweichend von der Darstellung in Figur 4 können im Zwischenraum zwischen der ersten Wand 60 und der zweiten Wand 70 Stege an der ersten Wand 60 angeordnet sein. Diese Stege sind insbesondere im Wesentlichen plattenförmig, radial und parallel zur Längsachse 28 ausgerichtet oder helikal ausgebildet. Ferner können die Stege tangential zur ersten Wand 60 angeordnet und ausgerichtet und/oder mit der zweiten Wand 70 verbunden sein.

Figur 5 zeigt eine schematische Darstellung eines Schnitts durch die in den Figuren 3 und 4 dargestellte Dichteinrichtung 30 entlang der in Figur 4 angedeuteten Ebene A-A senkrecht zur Längssachse 28.

Figur 5 zeigt die Dichteinrichtung 30 ohne ein eingeführtes medizinisches Instrument und somit in einem Zustand ähnlich dem der Darstellung der Figur 3. Die Dichtmembran 31 mit dem sternförmigen Schlitz 32 erscheint in Draufsicht. An den drei Enden der drei symmetrisch angeordneten radialen Abschnitte des Schlitzes 32 in der Dichtmembran 31 sind in den Figuren 1 bis 4 nicht dargestellte Verstärkungen 36 in Form kleiner Verdickungen 36 der Dichtmembran 31 vorgesehen. Die Verstärkungen 36 verhindern oder vermindern das Risiko eines Einreißens der Dichtmembran 31 an den Enden des Schlitzes 32 aufgrund der Kerbwirkung.

Von den Verstärkungen 36 abgesehen sind die dem Betrachter zugewandte proximale Oberfläche und die vom Betrachter abgewandte distale Oberfläche der Dichtmembran 31 im Wesentlichen planparallel. Die Dichtmembran 31 verschließt die Dichteinrichtung fluiddicht oder im Wesentlichen fluiddicht, indem sie im dargestellten entspannten Zustand eine im Wesentlichen ebene Platte senkrecht zur Längsachse und parallel zur Zeichenebene der Figur 5 bildet.

Figur 6 zeigt eine weitere schematische Darstellung der in den Figuren 3 bis 5 dargestellten Dichteinrichtung 30. Figur 6 ist eine Draufsicht, wobei die Zeichenebene parallel zur Schnittebene A-A der Figur 5 ist. Die Blickrichtung der Figur 6 ist entgegengesetzt zur Blickrichtung der Figur 5, das heißt Figur 6 zeigt die distale Seite der Dichteinrichtung 30.

Figur 6 zeigt die Dichteinrichtung 30 mit einem in die Dichteinrichtung 30 eingesetzten Schaft 24 eines medizinischen Instruments. Ähnlich wie in Figur 4 dargestellt, ist die Membran 31 elastisch verformt und liegt in drei durch den sternförmigen Schlitz 32 (siehe Figur 6) definierten Abschnitten am Umfang des Schafts 24 an. Die durch den sternförmigen Schlitz 32 gebildeten drei Teile der Dichtmembran 31 sind dabei aus der in Figur 4 erkennbaren ebenen Gestalt in die Gestalt von Ausschnitten eines Kreiszylindermantels verformt und liegen am äußeren Umfang des Schafts 24 an.

Durch die elastische Verformung der geschlitzten Dichtmembran 31 und des Übergangbereichs zwischen dem Rand der geschlitzten Dichtmembran 31 einerseits und der Ringdichtung 34 andererseits liegt die Ringdichtung 34 ähnlich wie eine herkömmliche O-RingDichtung im Wesentlichen linienförmig entlang einer kreisförmigen Linie, die den Schaft 24 des medizinischen Instruments umschließt, an diesem an und bildet eine fluiddichte Abdichtung.

In den Figuren 1, 2 und 3 ist ein Tubus mit nur einer Dichteinrichtung 30 dargestellt. Alternativ können an einem Tubus 20 mehrere Dichteinrichtungen 30 nebeneinander vorgesehen sein, damit mehrere medizinische Instrumente 22 gleichzeitig und nebeneinander fluiddicht in den Tubus eingeführt werden können. Dies kann mikroinvasive Eingriffe durch einen einzigen Zugang hindurch ermöglichen. Die Dichteinrichtungen 30 sind dazu insbesondere über ein elastisches Verbindungsstück mit dem proximalen Ende des starren Tubus des Tubus verbunden.

### Bezugszeichen

- 20: Tubus eines Trokars
- 22: medizinisches Instrument
- 24: Schaft des medizinischen Instruments
- 26: Bauchdecke eines Patienten
- 27: Hohlraum unter der Bauchdecke 26
- 28: Längsachse des Tubus 20
- 29: Richtung des Einführens eines medizinischen Instruments 22 in den Tubus 20
- 30: Dichteinrichtung
- 31: Dichtmembran der Dichteinrichtung 30
- 32: Schlitz in der Dichtmembran 31
- 33: Rand der Dichtmembran 31
- 34: Ringdichtung
- 36: Verstärkung an einem Ende des Schlitzes 32 in der Dichtmembran 31
- 39: Randprofil an der Dichteinrichtung 30
- 41: Lasche
- 60: erste elastische Wand
- 61: erster Rand der ersten Wand 60
- 62: zweiter Rand der ersten Wand 60
- 70: zweite elastische Wand
- 71: erster Rand der zweiten Wand 60
- 72: zweiter Rand der zweiten Wand 60
- 80: Übergangsbereich zwischen dem zweiten Rand 62 der ersten Wand 60 und dem ersten Rand 71 der zweiten Wand 70
- 81: Scheitel des Übergangsbereichs

## Patentansprüche

1. **Dichteinrichtung** (30) zur Abdichtung einer Durchführung für ein medizinisches Instrument (22), mit:
einem **Befestigungsbereich** (39), zur Befestigung der Dichteinrichtung (30) an einem Tubus (20) oder einer anderen medizinischen Vorrichtung;
einem **Dichtbereich** (31, 34), der zumindest entweder ausgebildet ist, um am Umfang eines in die Dichteinrichtung (30) eingeführten medizinischen Instruments (22) anzuliegen, oder ausgebildet ist, um die Dichteinrichtung (30) zu verschließen, wenn kein medizinisches Instrument (22) in die Dichteinrichtung (30) eingeführt ist,
einer ersten elastischen **Wand** (60), die den Dichtbereich ringartig umschließt, mit einem ringförmigen ersten Rand (61), der mit dem Dichtbereich (31, 34) verbunden ist, und einem ringförmigen zweiten Rand (62);
einer zweiten elastischen **Wand** (70), die die erste Wand (60) ringartigen umgibt und von dieser beabstandet ist, mit einem ringförmigen ersten Rand (71) und einem ringförmigen zweiten Rand (72), der mit dem Befestigungsbereich (39) verbunden ist;
einem ringförmigen **Übergangsbereich** (80), der den zweiten Rand (62) der ersten Wand (60) mit dem ersten Rand (71) der zweiten Wand (70) verbindet,
wobei der **Übergangsbereich** (80) eine **größere Wanddicke** aufweist als die erste Wand (60) und als die zweite Wand (70),
wobei die erste Wand (60) und die zweite Wand (70) parallel zueinander sind oder einen Winkel einschließen, der nicht größer als 20 Grad ist.

2. Dichteinrichtung (30) nach dem vorangehenden Anspruch, bei der die **größte Wanddicke des Übergangsbereichs** (80) um mindestens 50 Prozent größer als die minimale Wanddicke der zweiten Wand (70) ist.

3. Dichteinrichtung (30) nach einem der vorangehenden Ansprüche bei der die **Dicke der ersten Wand** (60) vom Dichtbereich (31, 34) zum Übergangsbereich (80) **zunimmt.**

4. Dichteinrichtung (30) nach einem der vorangehenden Ansprüche bei der die **Dicke der zweiten Wand** (70) vom Übergangsbereich (80) zum Befestigungsbereich (39) **zunimmt.**

5. Dichteinrichtung (30) nach einem der vorangehenden Ansprüche bei der der Übergangsbereich in einer Schnittebene, die eine Symmetrieachse der ersten Wand und der zweiten Wand enthält, einen im Wesentlichen **bogenförmigen** Querschnitt aufweist.

6. Dichteinrichtung (30) nach einem der vorangehenden Ansprüche, wobei
der Dichtbereich (31, 34) eine geschlitzte **Membran** (31) zum Verschließen der Dichteinrichtung, wenn kein medizinisches Instrument (22) in die Dichteinrichtung (30) eingeführt ist, und eine **Ringdichtung** (34), die ausgebildet ist, um am Umfang eines in die Dichteinrichtung (30) eingeführten medizinischen Instruments (22) anzuliegen, umfasst,
ein äußerer Rand (33) der geschlitzten **Membran** (31) bezogen auf die Einführrichtung (28, 29), in der ein medizinisches Instrument (22) in die Dichteinrichtung (30) einzuführen ist, **unmittelbar hinter** der **Ringdichtung** (34) angeordnet ist.

7. Dichteinrichtung (30) nach einem der vorangehenden Ansprüche, ferner mit:
einer **Verstärkung** (36) an einem Ende eines Schlitzes (32) in der Dichtmembran (31).

8. Dichteinrichtung (30) nach einem der vorangehenden Ansprüche, ferner mit:
einer **Lasche** (41) nahe dem Befestigungsbereich (39), zum manuellen Lösen einer Befestigung der Dichteinrichtung (30) an einem Tubus (20) oder einer anderen medizinischen Vorrichtung.

9. Dichteinrichtung (30) nach einem der vorangehenden Ansprüche wobei die Dichteinrichtung (30) **einstückig** ausgebildet ist.

10. Dichteinrichtung (30) nach einem der vorangehenden Ansprüche, ferner mit:
einer **reibungsmindernden Schicht** an einem Oberflächenbereich, der ausgebildet ist, um ein in die Dichteinrichtung (30) eingeführtes medizinisches Instrument (22) zu berühren.

11. **Tubus** (20) mit einer Dichteinrichtung (30) nach einem der vorangehenden Ansprüche.

12. Tubus (20) nach dem vorangehenden Anspruch, wobei der Tubus (20) außer der Dichteinrichtung (30) **keine weitere Dichteinrichtung** zum Verschluss des Tubus bei in den Tubus (20) eingesetztem medizinischem Instrument (22) oder zum Verschluss des Tubus (20) ohne eingesetztes medizinisches Instrument (22) aufweist.

## Claims

1. Sealing device (30) for sealing a passage for a medical instrument (22), with:
a fastening area (39) for fastening the sealing device (30) on a tube (20) or another medical appliance;
a sealing area (31, 34) which is at least either designed to bear on the circumference of a medical instrument (22) inserted into the sealing device (30) or is designed to close the sealing device (30) when no medical instrument (22) is inserted into the sealing device (30);
a first elastic wall (60) enclosing the sealing area in a ring shape, with an annular first edge (61), which is connected to the sealing area (31, 34), and an annular second edge (62);
a second elastic wall (70) which encloses the first wall (60) in a ring shape and is spaced apart therefrom, with an annular first edge (71) and an annular second edge (72), which is connected to the fastening area (39);
an annular transition area (80), which connects the second edge (62) of the first wall (60) to the first edge (71) of the second wall (70),
wherein the transition area (80) has a wall thickness greater than the first wall (60) and greater than the second wall (70),
wherein the first wall (60) and the second wall (70) are parallel to each other or enclose an angle not greater than 20 degrees.

2. Sealing device (30) according to the preceding claim, in which the greatest wall thickness of the transition area (80) is at least 50 percent greater than the minimum wall thickness of the second wall (70).

3. Sealing device (30) according to either of the preceding claims, in which the thickness of the first wall (60) increases from the sealing area (31, 34) to the transition area (80).

4. Sealing device (30) according to one of the preceding claims, in which the thickness of the second wall (70) increases from the transition area (80) to the fastening area (39).

5. Sealing device (30) according to one of the preceding claims, in which the transition area, in a sectional plane containing an axis of symmetry of the first wall and of the second wall, has a substantially arc-shaped cross section.

6. Sealing device (30) according to one of the preceding claims, wherein
the sealing area (31, 34) comprises a slit membrane (31) for closing the sealing device when no medical instrument (22) is inserted into the sealing device (30), and a ring seal (34) designed to bear on the circumference of a medical instrument (22) inserted into the sealing device (30),
an outer edge (33) of the slit membrane (31) is arranged directly behind the ring seal (34) with respect to the direction of insertion (28, 29) in which a medical instrument (22) is to be inserted into the sealing device (30).

7. Sealing device (30) according to one of the preceding claims, also with:
a reinforcement (36) at an end of a slit (32) in the sealing membrane (31).

8. Sealing device (30) according to one of the preceding claims, also with:
a tab (41) near the fastening area (39), for manually releasing the fastening of the sealing device (30) on a tube (20) or another medical appliance.

9. Sealing device (30) according to one of the preceding claims, wherein the sealing device (30) is configured in one piece.

10. Sealing device (30) according to one of the preceding claims, also with:
a friction-reducing layer on a surface area that is designed to touch a medical instrument (22) inserted into the sealing device (30).

11. Tube (20) with a sealing device (30) according to one of the preceding claims.

12. Tube (20) according to the preceding claim, wherein the tube (20) has, other than the sealing device (30), no further sealing device for closing the tube when a medical instrument (22) is inserted into the tube (20) or for closing the tube (20) without an inserted medical instrument (22).

## Revendications

1. Dispositif d'étanchéité (30) pour étanchéifier un passage pour un instrument médical (22), avec:
une zone de fixation (39), pour la fixation du dispositif d'étanchéité (30) à un tube (20) ou à un autre dispositif médical;
une zone d'étanchéité (31, 34), qui est réalisée soit pour s'appliquer à la périphérie d'un instrument médical (22) introduit dans le dispositif d'étanchéité (30) soit pour fermer le dispositif d'étanchéité (30) lorsqu'aucun instrument médical (22) n'est introduit dans le dispositif d'étanchéité (30),
une première paroi élastique (60), qui entoure en anneau la zone d'étanchéité, avec un premier bord annulaire (61), qui est relié à la zone d'étanchéité (31, 34), et un deuxième bord annulaire (62);
une deuxième paroi élastique (70), qui entoure en anneau la première paroi (60) et est espacée de celle-ci, avec un premier bord annulaire (71) et un deuxième bord annulaire (72), qui est relié à la zone de fixation (39);
une zone de transition annulaire (80), qui relie le deuxième bord (62) de la première paroi (60) au premier bord (71) de la deuxième paroi (70),
dans lequel la zone de transition (80) présente une plus grande épaisseur de paroi que la première paroi (60) et la deuxième paroi (70),
dans lequel la première paroi (60) et la deuxième paroi (70) sont parallèles l'une à l'autre ou forment un angle, qui n'est pas supérieur à 20 degrés.

2. Dispositif d'étanchéité (30) selon la revendication précédente, dans lequel la plus grande épaisseur de paroi de la zone de transition (80) est au moins 50 % plus élevée que l'épaisseur de paroi minimale de la deuxième paroi (70).

3. Dispositif d'étanchéité (30) selon l'une quelconque des revendications précédentes, dans lequel l'épaisseur de la première paroi (60) augmente de la zone d'étanchéité (31, 34) à la zone de transition (80).

4. Dispositif d'étanchéité (30) selon l'une quelconque des revendications précédentes, dans lequel l'épaisseur de la deuxième paroi (70) augmente de la zone de transition (80) à la zone de fixation (39).

5. Dispositif d'étanchéité (30) selon l'une quelconque des revendications précédentes, dans lequel la zone de transition présente, dans un plan de coupe qui contient un axe de symétrie de la première paroi et de la deuxième paroi, une section transversale essentiellement en forme d'arc.

6. Dispositif d'étanchéité (30) selon l'une quelconque des revendications précédentes, dans lequel
la zone d'étanchéité (31, 34) comprend une membrane fendue (31) pour fermer le dispositif d'étanchéité, lorsqu'aucun instrument médical (22) n'est introduit dans le dispositif d'étanchéité (30), et un joint annulaire (34), qui est configuré pour s'appliquer sur la périphérie d'un instrument médical (22) introduit dans le dispositif d'étanchéité (30),
un bord extérieur (33) de la membrane fendue (31) est disposé immédiatement derrière le joint annulaire (34), par rapport à la direction d'introduction (28, 29) dans laquelle un instrument médical (22) est introduit dans le dispositif d'étanchéité (30).

7. Dispositif d'étanchéité (30) selon l'une quelconque des revendications précédentes, avec en outre un renfort (36) à une extrémité d'une fente (32) dans la membrane d'étanchéité (31).

8. Dispositif d'étanchéité (30) selon l'une quelconque des revendications précédentes, avec en outre une patte (41) à proximité de la zone de fixation (39), pour le desserrage manuel d'une fixation du dispositif d'étanchéité (30) sur un tube (20) ou sur un autre dispositif médical.

9. Dispositif d'étanchéité (30) selon l'une quelconque des revendications précédentes, dans lequel le dispositif d'étanchéité (30) est réalisé en une seule pièce.

10. Dispositif d'étanchéité (30) selon l'une quelconque des revendications précédentes, avec en outre une couche diminuant le frottement sur une zone de surface, qui est formée afin de toucher un instrument médical (22) introduit dans le dispositif d'étanchéité (30).

11. Tube (20) avec un dispositif d'étanchéité (30) selon l'une quelconque des revendications précédentes.

12. Tube (20) selon la revendication précédente, dans lequel le tube (20) ne présente, hormis le dispositif d'étanchéité (30), aucun autre dispositif d'étanchéité pour la fermeture du tube lorsqu'un instrument médical (22) est introduit dans le tube (20) ou pour fermer le tube (20) sans instrument médical introduit (22).
